# FASCICULE DE BREVET EUROPEEN

(11) **EP 1 976 561 B1**
(45) Date de publication et mention de la délivrance du brevet: **17.08.2011**
(21) Numéro de dépôt: 07731510.9
(22) Date de dépôt: 24.01.2007
(51) Int. Cl.: A61K 47/26, A61K 47/12, A61K 47/02, A61P 31/00, A61K 31/5395

(54) **SOLUTION ANTI-INFECTIEUSE COMPRENANT UN COMPOSE DE TYPE PYRIDO(3,2,1-IJ)-BENZOXADIAZINE**
ANTIINFEKTIÖSE LÖSUNG MIT EINER PYRIDO(3,2,1-IJ)-BENZOXADIAZIN-VERBINDUNG
ANTI-INFECTIVE SOLUTION COMPRISING A COMPOUND OF PYRIDO(3,2,1-IJ)-BENZOXADIAZINE TYPE

(30) Priorité: 24.01.2006 FR 0650246
(43) Date de publication de la demande: 08.10.2008
(73) Titulaire: SA VETOQUINOL, 70200 Lure (FR)
(72) Inventeur: MOREAU, Marinette, F-70200 Saint Germain (FR)
(74) Mandataire: Novagraaf Technologies
(86) Numéro de dépôt international: PCT/FR2007/050682
(87) Numéro de publication internationale: WO 2007/085760

(56) Documents cités:
- US-A- 6 103 716
- ANONYMOUS: "Vetoquinol AG"[Online] septembre 2005 (2005-09), XP002444299 Extrait de l'Internet: URL:http://www.vetpharm.uzh.ch/reloader.ht m?http://www.vetpharm.uzh.ch/TAK/05000000/ 00055101.02?inhalt_c.htm> [extrait le 2007-07-26]

## Description

Le domaine technique de la présente invention concerne les solutions anti-infectieuses destinées à traiter les animaux.

La présente invention concerne plus précisément une solution anti-infectieuse comprenant au moins un composé pyrido(3,2,1-ij)-benzoxadiazine, et en particulier une solution concentrée.

Chez les animaux de rente, comme les bovins, porcins, caprins, ovins, volailles et/ou cheval, ainsi que chez les animaux de compagnie, l'administration, notamment par injection, de produits à visés thérapeutiques, prophylactiques ou métaphylactiques à visées anti-infectieuse est fréquente.

Le volume de produit administré par voie parentérale ainsi que le nombre de sites d'injection sont des critères importants dans le choix d'une formulation. Un objectif de la réalisation d'une formule concentrée est de diminuer le volume et/ou le nombre d'administrations, notamment en vue de réduire, pour les animaux de rente, les zones musculaires non valorisées à l'abattoir. Un autre critère important, qui peut également être pris en compte dans la réalisation de formules concentrées, est la tolérance locale, au site d'injection, notamment pour réduire les pertes à l'abattoir.

Cependant certains des produits anti-infectieux, par exemple la Marbofloxacine, présentent une faible solubilité dans l'eau. Ceci peut conduire à utiliser des technologies permettant d'augmenter leur solubilité, comme la salification, l'utilisation de complexes plus solubles que la molécule elle-même, par exemple en combinaison avec une cyclodextrine, de modificateurs de pH, de systèmes dispersés (émulsions, liposomes, systèmes vectoriels), ou l'ajout d'adjuvants tels les solvants organiques, les co-solvants, ou les tensioactifs.

Cependant, les techniques évoquées ci-dessus peuvent présenter une efficacité très relative par rapport à certains composés spécifiques.

Parmi les solutions commercialisées, on peut citer des solutions stables contenant jusqu'à 10 % de Marbofloxacine. Cependant, pour certaines indications thérapeutiques, prophylactiques ou métaphylactiques, notamment chez les animaux de rente, la dose thérapeutique est élevée. Ainsi l'emploi des solutions actuellement commercialisées nécessite l'administration de grands volumes d'injection, notamment supérieurs ou égaux à 40 ml, répartis en plusieurs sites d'injection. Ces conditions peuvent conduire à des problèmes de tolérance.

Le document EP 0 868 183 décrit des solutions aqueuses pharmaceutiques concentrées de danofloxacine comprenant des sels métalliques, dont la tolérance, au site d'injection, serait améliorée.

Cependant ce type de formulation, précédemment décrit pour les tétracyclines peut se révéler ne pas être applicable ou en tout cas ne pas être suffisamment efficace par rapport à tout type de molécule, en particulier en ce qui concerne certaines quinolones.

ANONYMOUS: "Vetoquinol AG"[Online] septembre 2005 (2005-09), XP002444299 Extrait de l'Internet: URL:http://www.vetpharm.uzh.ch/reloader.ht m?http://www.vetpharm.uzh.ch/TAK/05000000/00055101.02?inhalt-c.htm>
[extrait le 2007-07-26] décrit une solution injectable de marbofloxacine à 10% contenant de la gluconolactone. La solution contient en outre du crésol.

Or, pour différentes raisons, dont l'amélioration du bien être animal, un gain de temps, et/ou l'amélioration du rendement en viande, il peut être souhaitable de diminuer le volume de produit administré par voie parentérale, le nombre de sites d'injection et/ou d'améliorer la tolérance.

Il existe donc toujours un besoin pour des formules concentrées de composés anti-infectieux, en particulier de type quinolone, permettant de diminuer le volume, le nombre d'administrations et/ou présentant une tolérance améliorée, pouvant ainsi, pour les animaux de rente, diminuer les zones musculaires non valorisables.

L'invention a pour objet une solution comprenant i) de 10 à 30 % en poids par rapport au volume total de la solution d'au moins un composé de type pyrido(3,2,1-ij)-benzoxadiazine, ou au moins un de ses sels pharmaceutiquement acceptables, ii) au moins un agent de solubilisation ou agent solubilisant, (iii) de l'alcool benzylique en tant qu'agent stabilisant et un support pharmaceutiquement acceptable.

Par « composé de type pyrido(3,2,1-ij)-benzoxadiazine », on entend un composé répondant à la formule (I) suivante : 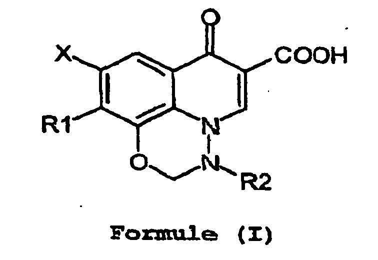 dans laquelle :
- X représente un atome d'hydrogène, d'halogène ou une fonction hydroxy, et en particulier un atome de fluor,
- R1 représente :
- un radical 1-pipérazinyle, qui peut être substitué en position 4 par un groupe méthyle, acétyle ou 4-aminobenzyle ;
- un radical morpholino ;
- un radical 1-pyrrolidinyle substitué en position 3 par un atome de chlore ou par un groupe amino, aminométhyle, (méthylamino) méthyle, (éthylamino) méthyle ou méthoxy ;
- un radical 1-imidazolyle qui peut être substitué en position 4 par le groupe méthyle ou
- un radical 1-pipéridyle substitué en position 4 par un groupe hydroxy ou méthoxy, et
- R2 représente un radical alkyle, linéaire, ramifié ou cyclique, comprenant de 1 à 10 atomes de carbone, notamment un groupe méthyle, et
ses sels pharmaceutiquement acceptables.

Le composé de type pyrido(3,2,1-ij)-benzoxadiazine peut tout particulièrement répondre à la formule (I) dans laquelle R1 représente un radical 1-pipérazinyle substitué en position 4 par un groupe méthyle, R2 représente un groupement méthyle et X représente un atome de fluor. Ce composé peut notamment être obtenu par les procédés décrits dans le document EP 0 259 804. Ce composé répond notamment à la Dénomination Commune Internationale de Marbofloxacine.

La solution comprend une teneur en composé de type pyrido(3,2,1-ij)-benzoxadiazine allant de 10 à 30 % en poids, notamment allant de 11 à 28 % en poids, en particulier allant de 12 à 27 % en poids, tout particulièrement allant de 13 à 25 % en poids, encore plus particulièrement allant de 14 à 23 % en poids, voire allant de 15 à 20 % en poids par rapport au volume total de la solution.

La solution comprend au moins un agent de solubilisation. Cet agent de solubilisation peut former un complexe avec le composés de type pyrido(3,2,1ij)-benzoxadiazine. En particulier lorsque cet agent de solubilisation est un acide, il peut former un sel avec le composé de type pyrido (3,2,1-ij)-benzoxadiazine.

La formation de tels complexes peut permettre d'améliorer la solubilisation du composé de type pyrido(3,2,1-ij)-benzoxadiazine en solution, et en particulier en solution aqueuse.

Des agents de solubilisation tels que
- les acides minéraux, notamment l'acide chlorhydrique, bromhydrique, sulfurique, phosphorique et nitrique, sont décrits Selon l'invention, l'agent de solubilisation est un acide organique, comme les acides carboxyliques les acides sulfoniques et les acides phosphoniques ; notamment l'acide formique, acétique, propionique, succinique, glycolique, lactique ou polylactique, malique, tartrique, citrique, ascorbique, maléfique, hydroxymaléique, phénylacétique, benzoïque, 4-aminobenzoïque, anthranilique, 4-hydroxybenzoïque, salicylique, aminosalicylique, nicotinique, méthane sulfonique, méthane sulfonique, hydroxyéthane sulfonique, benzène sulfonique, p-toluènesulfonique ; les acides polycarboxyliques, comme l'acide gluconique, l'acide glucuronique, l'acide galacturonique, l'acide isophtalique et l'acide lactobionique ; les acides aminés, comme l'acide aspartique et glutamique, la méthionine, le tryptophane, la lysine, l'arginine ; leurs esters, parmi lesquels on peut citer la gluconolactone ; et leurs sels, et
- leurs mélanges.

Parmi les agents de solubilisation des composés de type pyrido(3,2,1-ij)-benzoxadiazine, et en particulier la Marbofloxacine, on peut tout particulièrement citer l'acide lactique, l'acide gluconique et la gluconolactone.

La solution peut comprendre une teneur en agent de solubilisation allant de 4 à 58 % en poids, notamment de 5 à 35 % en poids, en particulier allant de 6 à 25 % en poids, voire allant de 7 à 20 % en poids par rapport au volume total de la solution.

Tout particulièrement, la solution selon l'invention comprend un rapport molaire agent de solubilisation/composé de type pyrido(3,2,1-ij)-benzoxadiazine allant de 0,9 à 4, notamment de 1 à 3, et en particulier de 1,1 à 2,4.

La solution peut comprendre une teneur en agent de solubilisation telle que le pH de la solution va de 2 à 7, notamnent de 2,8 à 5.

Les solutions décrites ci-dessus peuvent présenter une stabilité limitée, notamment lorsqu'elles comprennent une forte concentration en composé de type pyrido(3,2,1-ij)-benzoxadiazine et une faible concentration en agent de solubilisation.

Les inventeurs ont découvert qu'il était possible d'améliorer la faisabilité de certaines solutions concentrées et/ou leur stabilité par l'addition d'au moins un agent stabilisant.

Des agents stabilisants comme les alcools, les propylènes glycol, le polyéthylène glycol, la glycérine; plus particulièrement, un alcool, notamment aromatique, alkyle, arylalkyle ou alkylaryle, en particulier comprenant de 5 à 15 atomes de carbone, sont décrits. Selon l'invention, l'agent stabilisant est l'alcool benzylique et ses dérivés.

On entend par « dérivé d'alcool benzylique » au sens de la présente invention des composés répondant à la formule (II): 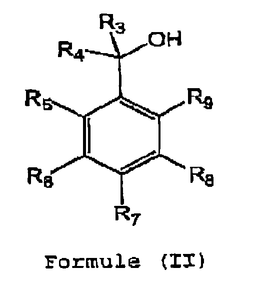 dans laquelle R₃, R₄, R₅, R₆, R₇, R₈ et R₉ représentent indépendamment un atome d'hydrogène, d'halogène, une fonction alcool, éther, amine, éventuellement substituée, thioéther, ester, amide, éventuellement substituée, un radical alkyle, aryle, aralkyle, alkaryle.

En particulier, la solution comprend une teneur en agent stabilisant allant de 0,2 à 20 % en poids, notamment de 0,3 à 10% en poids, en particulier de 0,4 à 5 % en poids, et tout particulièrement de 0,5 à 3 % en poids par rapport au volume total de la solution.

D'autre part, la solution peut comprendre un rapport molaire agent stabilisant/composé de type pyrido(3,2,1-ij)-benzoxadiazine allant de 0,02 à 7, notamment de 0,05 à 5, en particulier de 0,1 à 1,5, voire de 0,2 à 1.

En particulier, les solutions peuvent comprendre un rapport molaire agent solubilisant/agent stabilisant allant de 1 à 15, notamment de 1,5 à 10, en particulier de 2 à 8, voire de 2,5 à 7.

Les solutions selon l'invention peuvent en outre comprendre au moins un additif, notamment choisi dans le groupe comprenant :
- un solvant, par exemple choisi parmi les solvants non aqueux utilisables par voie parentérale i.e. les solvants non miscibles à l'eau, par exemple les huiles végétales, l'oléate d'éthyle, ou les solvants miscibles à l'eau, notamment les alcools, comme l'éthanol ; les amides, comme le N,N diméthylacétamide ; les esters de polyol, comme le glycéride polyglucosé ; les éthers, comme le diméthyl-1,3-dioxolane-4-méthanol, l'éther monoéthylique du diéthylène glycol, le glycérol formal; le polyéthylène glycol, notamment PEG 300 et PEG 400 ; les polyols, comme le glycérol, le propylène glycol ; le diméthylsulfoxyde ; le 2-pyrrolidone ; ou un mélange de ceux-ci,
- un tensioactif, par exemple non ionique, anionique, cationique, ou un mélange de tensioactif, et tout particulièrement un tensioactif non ionique,
- un agent chélatant, par exemple l'EDTA
- un conservateur, notamment un antioxydant, ou un antimicrobien, et
- un mélange de ceux-ci.

La solution peut en particulier être une solution aqueuse.

Le pH de la solution peut aller de 2 à 7, notamment de 2,8 à 5.

Tout particulièrement, la solution selon l'invention est sous forme d'une solution injectable, en particulier par voie intramusculaire, sous-cutanée, intrapéritonéale et/ou intraveineuse. Ladite solution peut encore être une infusion ou une perfusion. Cette solution peut être destinée aux animaux dits de rente, comme les ovins, bovins, porcins, caprins, chevaux et/ou volailles, et/ou aux animaux de compagnie, comme les chiens et les chats.

Tout particulièrement, la solution présente une viscosité allant de 10⁻⁴ à 10⁻², en particulier de 10⁻³ à 5.10⁻³ Pa.s à 20°C.

La solution peut être limpide, en particulier ne pas comprendre de particules en suspension.

Selon un autre de ses aspects, l'invention a pour objet l'utilisation d'au moins un composé de type pyrido(3,2,1-ij)-benzoxadiazine tel que décrit ci-dessus dans la préparation d'une solution destinée à traiter ou à prévenir des infections, en particulier chez l'animal, ledit composé de type pyrido(3,2,1-ij)-benzoxadiazine étant présent en une teneur allant de 10 à 30 % en poids, notamment 11 à 28 % en poids, voire de 12 à 27 % en poids, en particulier de 13 à 25 % en poids, tout particulièrement de 14 à 23 % en poids, voire de 15 à 20 % en poids par rapport au volume total de la solution.

Selon encore un autre de ses aspects, l'invention a encore pour objet l'utilisation d'une solution selon l'invention pour la préparation d'un médicament destiné à traiter ou à prévenir des infections, en particulier chez l'animal.

Parmi les infections susceptibles d'être traitées, on peut citer les infections du système respiratoire, du système de reproduction, du système urinaire, du système digestif, du système locomoteur, cardiovasculaire, du type cutanée, otite, ophtalmique, notamment chez les animaux.

Selon un autre de ses aspects, l'invention a pour objet une méthode de traitement, de prévention et de métaphylaxie des infections chez des animaux. Ladite méthode de traitement peut comprendre l'injection de 0,01 à 0,5 ml de solution concentrée par kg, notamment de 0,01 à 0,1 ml/kg d'animal à traiter. En particulier le traitement peut comprendre une seule injection en un seul point.
Par exemple pour un animal de 300 kg, le traitement d'une infection respiratoire peut être réalisé avec « MARBOCYL S » avec un volume de 24 ml, administré en 2 points d'injection, et avec la solution invention, avec un volume de 15 ml, injecté en 1 seul point.
Par « traitement » au sens de la présente invention, on entend le traitement en lui-même, mais aussi la prophylaxie et la métaphylaxie.

utilisation d'au moins un composé choisi parmi les alcools, les propylènes glycol, le polyéthylène glycol, la glycérine, en tant qu'agent stabilisant une solution d'un composé de Formule (I) est décrite dans le présent document. Par exemple, il est décrit que, l'agent stabilisant est un alcool, notamment aromatique, alkyle, arylalkyle ou alkylaryle, en particulier comprenant de 5 à 15 atomes de carbone. Selon l'invention, l'alcool benzylique et ses dérivés sont utilisés en tant qu'agent stabilisant d'une solution

comprenant au moins un composé de formule (I), en particulier à une forte concentration, notamment supérieure à 10 % en poids par rapport au volume total de la solution, et un agent de solubilisation.

L'utilisation d'au moins un composé choisi parmi :
- les acides minéraux, notamment l'acide chlorhydrique, bromhydrique, sulfurique, phosphorique et nitrique, et
- les acides organiques, comme les acides carboxyliques, les acides sulfoniques et les acides phosphoniques ; notamment l'acide formique, acétique, propionique, succinique, glycolique, lactique ou polylactique, malique, tartrique, citrique, ascorbique, maléique, hydroxymaléique, phénylacétique, benzoïque, 4-aminobenzoïque, anthranilique, 4-hydroxybenzoïque, salicylique, aminosalicylique, nicotinique, méthane sulfonique, éthane sulfonique, hydroxyéthane sulfonique, benzène sulfonique, p-toluènesulfonique ; les acides polycarboxyliques, comme l'acide gluconique, l'acide glucuronique, l'acide galacturonique, l'acide isophtalique et l'acide lactobionique ; les acides aminés, comme l'acide aspartique et glutamique, la méthionine, le tryptophane, la lysine, l'arginine ; leurs esters, parmi lesquels on peut citer la gluconolactone ; et leurs sels, et
- leurs mélanges,
en tant qu'agent de solubilisation d'une solution d'un composé de Formule (I), en particulier à une forte concentration, notamment supérieure à 10 % en poids par rapport au volume total de la solution, comprenant éventuellement en outre un agent stabilisant est décrite.

L'utilisation d'un agent stabilisant, et en particulier d'alcool benzylique, peut permettre en outre aux solutions concentrées en composé de formule (I), notamment en Marbofloxacine, et comprenant au moins un agent de solubilisation, notamment de la gluconolactone, de présenter un pH et/ou une osmolarité moindre par rapport aux solutions comprenant une concentration équivalente en composé de formule (I) et éventuellement en agent de solubilisation. Ceci peut en particulier permettre aux solutions concentrées comprenant au moins un composé de formule (I), un agent de solubilisation et un agent stabilisant de présenter un pH et/ou une osmolarité proche des compositions moins concentrées, notamment en composé de formule (I) et/ou en agent de solubilisation. Ceci est lié à la présence d'un agent stabilisant qui peut permettre d'utiliser moins d'agent de solubilisation.
En d'autres termes, l'addition d'agent stabilisant rend également possible la fabrication de certaines solutions très concentrées en composé de formule (I) adaptées à l'injection, qui ne le seraient pas, ou qui le seraient difficilement, sans l'ajout de cet agent. Effectivement, l'effet stabilisant peut augmenter avec la teneur en agent stabilisant.

Tout particulièrement, la présente invention a pour objet l'utilisation de l'association d'une part d'alcool benzylique et d'autre part de l'acide lactique, l'acide gluconique et/ou la gluconolactone en tant qu'agent solubilisant et stabilisant d'une solution de composé de formule (I), en particulier à une concentration supérieure à 10 %, notamment supérieure à 11 %, en particulier supérieure à 12 % en poids, tout particulièrement supérieure à 13 % en poids, voire supérieure à 14 % en poids par rapport au volume de la solution.

Les exemples suivants sont donnés à titre illustratif .

### EXEMPLES

### Exemple 1 :

Les solutions décrites dans le tableau 1 suivant ont été préparées.

| | **Lot 1** | **Témoin** |
|---|---|---|
| Marbofloxacine (en gramme) | 15,00 | 10,00 |
| Gluconolactone (en gramme) | 8,11 | 8,00 |
| Eau déminéralisée qsp | 100 ml | 100 ml |

Des porcs de 70-80 Kg ont reçu une injection unique de solution témoin dans l'encolure droite et d'une solution du lot 1 dans l'encolure gauche, par voie intramusculaire, à la dose de 8 mg/Kg. La tolérance locale (lésions post-mortem) a été examinée 1 semaine après administration.
L'injection avec le lot 1 présente un volume de lésion d'environ 9 cm³ alors que l'injection avec le témoin montre un volume de lésion de 32 cm³.
Ceci démontre une meilleure tolérance locale de la solution concentrée en Marbofloxacine, par rapport à la solution témoin.

### Exemple 2 :

Les lots suivants ont été préparés :

| | Lot 1 | Lot 2 | Lot 3 | Lot 4 |
|---|---|---|---|---|
| Marbofloxacine | 20,00 g | 20,00 g | 20,00 g | 20,00 g |
| Gluconolactone | 19,00 g | 19,00 g | 17,00 g | 17,00 g |
| Alcool benzylique | - | 2,00 g | - | 2,00 g |
| Eau déminéralisée | qsp 100 ml | qsp 100 ml | qsp 100 ml | qsp 100 ml |
| **Stabilité à froid** | **24 heures** | **> 7 jours** | **24 heures** | **> 7 jours^{*}** |

| | | | | |
|---|---|---|---|---|
| ^{*} Après 7 jours, aucun précipité n'a été observé | | | | |

Par « stabilité à froid », on entend le temps pour qu'il se forme un précipité lorsque les lots sont laissés immobiles entre +4 et +8°C.

Ceci démontre bien une amélioration de la stabilité des solutions selon l'invention.

## Revendications

1. Solution anti-infectieuse comprenant :
(i) de 10 à 30 % en poids par rapport au volume total de la solution d'au moins un composé répondant à la formule (I) suivante : 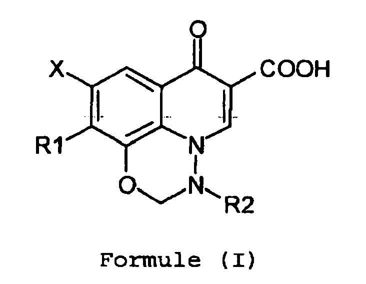
- X représente un atome d'hydrogène, d'halogène ou une fonction hydroxy,
- R1 représente :
- un radical 1-pipérazinyle, qui peut être substitué en position 4 par un groupe méthyle, acétyle ou 4-aminobenzyle ;
- un radical morpholino ;
- un radical 1-pyrrolidinyle substitué en position 3 par un atome de chlore ou par un groupe amino, aminométhyle, (méthylamino) méthyle, (éthylamino) méthyle ou méthoxy ;
- un radical 1-imidazolyle qui peut être substitué en position 4 par le groupe méthyle ou
- un radical 1-pipéridyle substitué en position 4 par un groupe hydroxy ou méthoxy, et
- R2 représente un radical alkyle, linéaire, ramifié ou cyclique, comprenant de 1 à 10 atomes de carbone, ou au moins un de ses sels pharmaceutiquement acceptables,
(ii) de l'alcool benzylique en tant qu'agent stabilisant, et
(iii) au moins un agent de solubilisation choisi dans le groupe comprenant les acides organiques et leurs esters, dans un support pharmaceutiquement acceptable.

2. Solution selon la revendication 1 dans laquelle le composé répond à la formule (I) dans laquelle R1 représente un radical 1-pipérazinyle substitué en position 4 par un groupe méthyle, R2 représente un groupement méthyle et X représente un atome de fluor.

3. Solution selon la revendication 1 ou 2, laquelle comprend une teneur en composé de formule (I) allant de 11 à 28 % en poids par rapport au volume total de la solution.

4. Solution selon l'une quelconque des revendications 1 à 3, dans laquelle l'agent de solubilisation est choisi dans le groupe comprenant l'acide lactique, l'acide gluconique et la gluconolactone.

5. Solution selon l'une quelconque des revendications 1 à 4, laquelle comprend une teneur en agent de solubilisation allant de 4 à 58 % en poids, par rapport au volume total de la solution.

6. Solution selon l'une quelconque des revendications 1 à 5, dans laquelle le rapport molaire agent de solubilisation/composé de formule (I) va de 0,9 à 4.

7. Solution selon l'une quelconque des revendications 1 à 6, dans laquelle l'agent de solubilisation est présent en une teneur telle que le pH de la solution va de 2 à 7.

8. Solution selon l'une quelconque des revendications 1 à 7, laquelle comprend une teneur en agent stabilisant allant de 0,2 à 20 % en poids par rapport au volume total de la solution.

9. Solution selon l'une quelconque des revendications 1 à 8, laquelle comprend un rapport molaire agent stabilisant/composé de formule (I) allant de 0,02 à 7.

10. Solution selon l'une quelconque des revendications 1 à 9, laquelle présente un pH allant de 2 à 7.

11. Solution selon l'une quelconque des revendications 1 à 10 pour utilisation comme médicament anti-infectieux.

12. Utilisation d'une solution selon l'une quelconque des revendications 1 à 11 pour l'obtention d'un médicament destiné à traiter ou à prévenir des infections chez l'animal.

## Claims

1. An anti-infection solution comprising:
(i) from 10% to 30% by weight with respect to the total volume of the solution of at least one compound of the following formula (I): 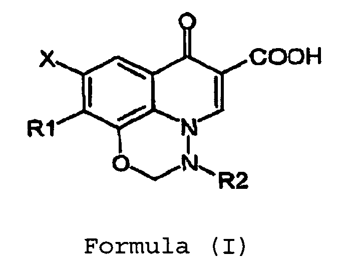
- X represents a hydrogen or halogen atom or a hydroxyl functional group,
- R1 represents:
a 1-piperazinyl radical, which may be substituted in position 4 by a methyl, acetyl or 4-aminobenzyl group;
a morpholino radical:
a 1-pyrrolidinyl radical substituted in position 3 by a chlorine atom or by an amino, aminomethyl, (methylamino) methyl, (ethylamino) methyl or methoxy group;
a 1-imidazolyl radical that can be substituted in position 4 by a methyl group, or
a 1-piperidyl radical substituted in position 4 by a hydroxyl or methoxy group, and
- R2 represents a linear, branched or cyclic alkyl radical, comprising 1 to 10 carbon atoms, or
at least one of its pharmaceutically acceptable salts,
(ii) benzyl alcohol as a stabilising agent, and
(iii) at least one solubilisation agent chosen from the group comprising organic acids and their esters, in a pharmaceutically acceptable carrier.

2. A solution according to claim 1 wherein the compound has formula (I), wherein R1 represents a 1-piperazinyl radical substituted in position 4 by a methyl group, R2 represents a methyl group and X represents a fluorine atom.

3. A solution according to claim 1 or 2, which comprises a proportion of compound of formula (I) ranging from 11% to 28% by weight with respect to the total volume of the solution.

4. A solution according to any one of claims 1 to 3, wherein the solubilisation agent is chosen from the group comprising lactic acid, gluconic acid and gluconolactone.

5. A solution according to any one of claims 1 to 4, which comprises a proportion of solubilisation agent ranging from 4% to 58% by weight, with respect to the total volume of the solution.

6. A solution according to any one of claims 1 to 5, wherein the mole ratio between solubilisation agent and compound of formula (I) ranges from 0.9 to 4.

7. A solution according to any one of claims 1 to 6, wherein the solubilisation agent is present in a proportion such that the pH of the solution ranges from 2 to 7.

8. A solution according to any one of claims 1 to 7, which comprises a proportion of stabilising agent ranging from 0.2 to 20% by weight with respect to the total volume of the solution.

9. A solution according to any one of claims 1 to 8, which comprises a mole ratio between stabilising agent and compound of formula (I) ranging from 0.02 to 7.

10. A solution according to any one of claims 1 to 9, which has a pH ranging from 2 to 7.

11. A solution according to any one of claims 1 to 10 for use as an anti-infection medicament.

12. Use of a solution according to any one of claims 1 to 11 for procuring a medicament intended to treat or prevent infections in animals.

## Patentansprüche

1. Eine antiinfektiöse Lösung, die folgendes enthält:
(i) 10 bis 30 Gewichtsprozente in Bezug auf das Gesamtvolumen der Lösung von zumindest einer Verbindung entsprechend der folgenden Formel (I): 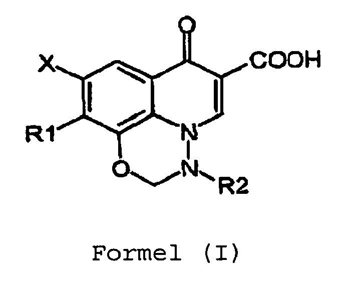
- X steht für ein Wasserstoff- oder Halogenatom oder eine Hydroxygruppe,
- R1 steht für:
- ein 1-Piperazinyl-Radikal, das in Position 4 durch eine Methyl-, Acetyl- oder 4-Aminobenzylgruppe substituiert sein kann;
- ein Morpholino-Radikal;
- ein 1-Pyrrolidinyl-Radikal, das in Position 3 durch ein Chloratom oder durch eine Amino-, Aminomethyl, (Methylamino) Methyl-, (Ethylamino) Methyl- oder Methoxygruppe substituiert sein kann;
- ein 1-Imidazolyl-Radikal, das in Position 4 durch eine Methylgruppe substituiert sein kann, oder
- ein 1-Piperidyl-Radikal, das in Position 4 durch eine Hydroxy- oder Methoxy-Gruppe substituiert ist, und
- R2 steht für eine lineares, verzweigtes oder zyklisches Alkyl-Radikal, das 1 bis 1O Kohlenstoffatome enthält, oder zumindest eines seiner pharmazeutisch akzeptablen Salze,
(ii) Benzylalkohol als Stabilisierungsmittel, und
(iii) zumindest ein Solubilisierungsmittel, das innerhalb der Gruppe gewählt wird, zu der organische Säuren und ihre Ester in einem pharmazeutisch akzeptablen Träger gehören.

2. Lösung nach Anspruch 1, in der die Verbindung der Formel (I) entspricht, wobei R1 für ein 1-Piperazinyl-Radikal steht, das in Position 4 durch eine Methylgruppe substituiert ist, R2 steht für eine Methylgruppe und X steht für ein Fluoratom.

3. Lösung nach Anspruch 1 oder 2, die einen Anteil der Verbindung gemäß Formel (I) enthält, der zwischen 11 und 28 Gewichtsprozenten in Bezug auf das Gesamtvolumen der Lösung liegt.

4. Lösung nach einem der Ansprüche 1 bis 3, wobei das Solubilisierungsmittel innerhalb der Gruppe gewählt wird, zu der Milchsäure, Gluconsäure und Gluconolacton gehören.

5. Lösung nach einem der Ansprüche 1 bis 4, die einen Anteil des Solubilisierungsmittels enthält, der zwischen 4 und 58 Gewichtsprozenten in Bezug auf das Gesamtvolumen der Lösung liegt.

6. Lösung nach einem der Ansprüche 1 bis 5, wobei das Molverhältnis zwischen dem Solubilisierungsmittel und der Verbindung gemäß Formel (I) zwischen 0,9 und 4 liegt.

7. Lösung nach einem der Ansprüche 1 bis 6, wobei das Solubilisierungsmittel in solch einem Anteil vorliegt, dass der pH-Wert der Lösung zwischen 2 und 7 liegt.

8. Lösung nach einem der Ansprüche 1 bis 7, die einen Anteil des Stabilisierungsmittels enthält, der zwischen 0,2 und 20 Gewichtsprozenten in Bezug auf das Gesamtvolumen der Lösung liegt.

9. Lösung nach einem der Ansprüche 1 bis 8, wobei das Molverhältnis zwischen dem Stabilisierungsmittel und der Verbindung gemäß Formel (I) zwischen 0,02 und 7 liegt.

10. Lösung nach einem der Ansprüche 1 bis 9, die einen pH-Wert von 2 bis 7 aufweist.

11. Lösung nach einem der Ansprüche 1 bis 10 zur Verwendung als antiinfektiöses Medikament.

12. Verwendung einer Lösung nach einem der Ansprüche 1 bis 11 zum Erhalt eines Medikaments zur Behandlung oder Vorbeugung von Infektionen bei Tieren.
